# FASCICULE DE BREVET EUROPEEN

(11) **EP 0 666 251 B1**
(45) Date de publication et mention de la délivrance du brevet: **04.12.1996**
(21) Numéro de dépôt: 95400063.4
(22) Date de dépôt: 12.01.1995
(51) Int. Cl.: C07C 271/16, A61K 7/06

(54) **Nouveaux N-(hydroxyalkyl) carbamates d'alkyle et leurs applications en cosmétique, plus particulièrement dans des compositions capillaires**
N-(Hydroxyalkyl)-substituierte Alkylcarbamate und ihre Verwendung in der Kosmetik, insbesondere in Haarpflegemitteln
N-hydroxy substituted alkyl carbamates and their use in cosmetics especially in hair-care compositions

(30) Priorité: 07.02.1994 FR 9401341
(43) Date de publication de la demande: 09.08.1995
(73) Titulaire: L'OREAL, 75008 Paris (FR)
(72) Inventeur: Mahieu, Claude, F-75017 Paris (FR); Bollens, Eric, F-94410 Saint Maurice (FR); Cauwet, Danièle, F-75011 Paris (FR)
(74) Mandataire: Dodin, Catherine

(56) Documents cités:
- EP-A- 0 420 722
- EP-A- 0 450 527
- EP-A- 0 577 506
- US-A- 2 808 402

## Description

La présente invention a pour objet de nouveaux N-(hydroxyalkyl) carbamates d'alkyle et leur procédé de préparation. Elle a également pour objet des compositions cosmétiques contenant ces composés. Elle a encore plus particulièrement pour objet l'utilisation de telles compositions pour le traitement des cheveux.

Des composés de formule R₁-CHOH- CH(CH₂OH)-NH-CO-R₂ ont été décrits dans la demande EP-A-0 420 722.

La demande EP-A-0 577 506 décrit l'utilisation de carbamates d'aminopolyols en tant qu'épaississants dans des solutions de tensioactifs.

La présente invention a donc pour objet les composés de formule générale (I) suivante : 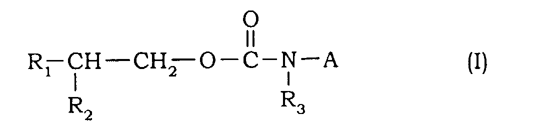 dans laquelle:
- R₁ représente un radical alkyle ou alkylène ayant environ de 4 à 18 atomes de carbone,
- R₂ représente un radical alkyle ou alkylène ayant environ de 2 à 16 atomes de carbone ,
- R₃ représente un atome d'hydrogène ou un radical alkyle ayant environ de 1 à 6 atomes de carbone,
- A représente un groupement hydrophile non-ionique.

De préférence, R₁ représente un radical alkyle ayant de 4 à 14 atomes de carbone, R₂ représente un radical alkyle ayant de 2 à 12 atomes de carbone, R₃ représente un atome d'hydrogène ou un radical méthyle.

De préférence, A représente un radical : 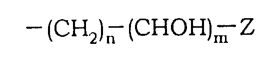 dans lequel n représente un nombre entier égal à 0 ou 1, m représente un nombre entier compris entre 0 et 5, et Z est un radical alkyle monohydroxylé ou polyhydroxylé ayant de 1 à 4 atomes de carbone. En particulier, Z est choisi parmi le groupe comprenant les radicaux suivants:

-CH₂OH -CH₂-CH₂OH -CH(CH₂OH)₂

-C(CH₂OH)₃

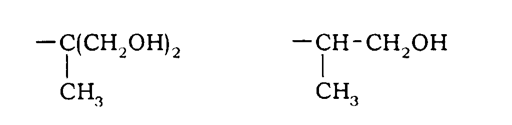

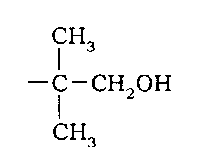

De préférence, Z est choisi parmi le groupe comprenant les radicaux

-CH₂OH -C(CH₂OH)₃

Parmi les composés préférés correspondant à la formule générale (I), on peut notamment citer :
- le N-2-éthylhexyloxycarbonyl-N-méthyl-D-glucamine
- le N-2-hexyldécyloxycarbonyl-N-méthyl-D-glucamine
- le N-2-butyloctyloxycarbonyl-N-méthyl-D-glucamine
- le N-2-décyltétradécyloxycarbonyl-N-méthyl-D-glucamine
- le N-2-dodécylhexadécyloxycarbonyl-N-méthyl-D-glucamine
- le N-2-décyltétradécyloxycarbonyl-D-glucamine
- le N-2-hexyldécyloxycarbonyl-D-glucamine
- le 3-N-(2-décyltétradécyloxycarbonyl)-amino-1,2-propanediol
- le 2-N-(2-décyltétradécyloxycarbonyl)-amino-2-hydroxyméthyl-1,3-propanediol
- le 2-N-(2-hexyldécyloxycarbonyl)-amino-1-éthanol, et
- le 2-N-(2-décyltétradécyloxycarbonyl)-amino-1-éthanol.

La présente invention a également pour objet le procédé de préparation des composés de formule (I). Ce procédé consiste à faire réagir dans un solvant un amino alcool de formule R₃-NH-A avec un composé de formule (II) : 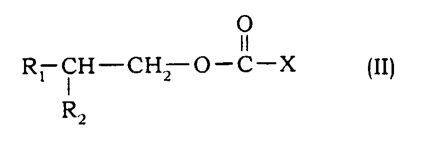 R₁, R₂, R₃, A ayant les même significations que celles données ci-dessus et X représentant un atome d'halogène, notamment un atome de chlore, un radical dérivé d'un azole, notamment un radical provenant d'un imidazole tel que celui de formule (III) :

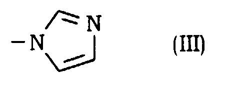

Comme solvant, on peut utiliser le dichlorométhane, le dichloro-1,2 éthane, le trichloro-1,1,1 éthane, le chloroforme, l'acétonitrile, le toluène, le dioxane, le tétrahydrofurane, le diméthoxy-1,2 éthane, le cyclohexane, l'eau ou un mélange de ces solvants.

La réaction est effectuée à une température comprise entre -5°C et 50°C, de préférence inférieure à 10°C.
La réaction peut être effectuée en présence d'une base. Celle-ci peut être choisie parmi les hydroxydes de métaux alcalins ou alcalino-terreux, l'hydrogénocarbonate de sodium, les alcoolates de métaux alcalins, les hydrures alcalins, les amines tertiaires telles que la pyridine ou la triéthylamine. On utilise de préférence l'hydrogénocarbonate de sodium.

La présente invention a également pour objet une composition cosmétique comprenant, dans un véhicule cosmétiquement acceptable, au moins un composé de formule (I) tel que défini ci-dessus. Plus particulièrement mais non exclusivement, la composition cosmétique est une composition capillaire.

Il est bien connu que les cheveux sont sensibilisés ou fragilisés à des degrés divers par l'action des agents atmosphériques, ainsi que par l'action répétée des différents traitements capillaires tels que les permanentes, le défrisage, la teinture, la décoloration. Les cheveux deviennent alors rêches au toucher, perdent leur aspect brillant. Ils sont difficiles à démêler et à coiffer.

Il est donc nécessaire dans le domaine du traitement des cheveux de mettre en oeuvre dans des produits capillaires des agents de traitement dans le but de réparer les dommages subis par le cheveu. Ces agents de traitement doivent apporter d'excellentes propriétés au cheveu telles que démêlage, brillance, nervosité et toucher agréable.

La demanderesse a maintenant découvert, de façon inattendue et surprenante, que les composés nouveaux de formule (I) présentent d'excellentes propriétés : utilisés dans des compositions capillaires, ils confèrent aux cheveux des effets coiffant, lissant et gainant et facilitent leur démêlage. Ils offrent donc un interêt tout particulier pour le traitement des cheveux.

Dans les compositions selon l'invention, les composés de formule (I) sont présents à une concentration comprise entre 0,001 et 15 % en poids et de préférence entre 0,1 et 10% en poids par rapport au poids total de la composition.

Les compositions peuvent se présenter sous forme de lotion aqueuse ou hydroalcoolique, monophasique ou polyphasique, de gel monophasique ou polyphasique, d'émulsion, de crème, de dispersion vésiculaire, de mousse, de spray.

Les compositions capillaires peuvent se présenter sous forme de shampooing, d'après-shampooing à rincer ou non, de compositions pour permanente, défrisage, coloration ou décoloration, ou encore sous forme de compositions à rincer, à appliquer avant ou après une coloration, une permanente ou un défrisage ou encore entre les deux étapes d'une permanente ou d'un défrisage.

Les compositions peuvent par ailleurs contenir des additifs cosmétiques conventionnels choisis parmi les corps gras, les solvants organiques, les silicones, les épaississants, les adoucissants, les tensio-actifs, les polymères anioniques, cationiques, non-ioniques ou amphotères, les agents anti-mousses, les agents conditionneurs du cheveu tels que des protéines, des vitamines, les agents traitants (agents anti-chute, antipelliculaires), les colorants, les parfums, les conservateurs, les agents propulseurs.

Plus précisément, comme corps gras, on peut utiliser une huile ou une cire ou leur mélange, des acides gras, des alcools gras, des esters d'acides gras tels que les triglycérides d'acide gras en C₆ à C₁₈, de la vaseline, de la paraffine, de la lanoline, de la lanoline hydrogénée ou acétylée.

Parmi les huiles, on peut citer les huiles minérales, animales, végétales ou les huiles de synthèse, et notamment l'huile de vaseline, de paraffine, de ricin, de jojoba, de sésame, ainsi que les huiles et les gommes de silicone et les isoparaffines.

Parmi les cires, on peut citer les cires animales, végétales, minérales ou de synthèse, et notamment les cires d'abeilles, de Candelila, les ozokérites, les cires microcristallines ainsi que les cires et résines de silicone.

Parmi les solvants organiques usuellement utilisés dans les compositions cosmétiques, on peut citer plus précisément les mono-alcools ou polyalcools inférieurs en C₁ à C₆ comme l'éthanol, l'isopropanol, l'éthylèneglycol, le diéthylènegycol, le propylèneglycol, le glycérol.

Les agents épaississants peuvent être choisis notamment parmi l'alginate de sodium, la gomme arabique, les dérivés cellulosiques tels que la méthylcellulose, l'hydroxyméthylcellulose, l'hydroxyéthylcellulose, l'hydroxypropylcellulose, l'hydroxypropylméthylcellulose, la gomme de guar ou ses dérivés, la gomme de xanthane, les scléroglucanes, les acides polyacryliques réticulés.

Comme agents tensio-actifs et comme polymères , on peut utiliser tous ceux bien connus de l'état de la technique notamment pour leur utilisation dans des compositions capillaires.

Les compositions peuvent se présenter sous forme de dispersion vésiculaire de lipides amphiphiles ioniques ou non-ioniques. Elles sont préparées notamment en faisant gonfler les lipides dans une solution aqueuse pour former des sphérules dispersées dans le milieu aqueux comme décrit dans STANDISH & WATKINS, J. Mol. Biol., 13,238 (1965) ou dans les brevets FR-A-2.315.991 et FR-A-2.416.008 de la demanderesse. Les différents types de procédés de préparation sont décrits dans "Les liposomes en biologie cellulaire et pharmacologie", Edition INSERM/John Libery Eurotext, 1987, pages 6 à 18.

Le pH des compositions selon l'invention est généralement compris entre 4 et 8 et de préférence entre 5 et 7.

L'invention a également pour objet l'utilisation d'une composition telle que définie ci-dessus pour le traitement des cheveux ainsi qu'un procédé de traitement des cheveux consistant à appliquer sur les cheveux une composition telle que définie ci-dessus.

On va maintenant donner à titre d'illustration et sans aucun caractère limitatif plusieurs exemples de préparation de composés selon l'invention ainsi que des exemples de compositions cosmétiques les contenant.

### Exemple 1 : Préparation de la N-2-éthylhexyloxycarbonyl-N-méthyl-D-glucamine

Dans un réacteur, on dissout 117 g (0,6 mole) de N-méthyl-D-glucamine dans un mélange de 800 ml d'eau et 400 ml de tétrahydrofurane, puis on disperse 201,6 g (2,4 moles) d'hydrogénocarbonate de sodium.

En maintenant la température du mélange réactionnel à 5° C, on ajoute goutte-à-goutte 115,6 g (0,6 mole) de chloroformiate de 2-éthylhexyle puis on laisse réagir 3 heures sous agitation à 5° C, une nuit au repos à la température ambiante.

Le mélange réactionnel est alors filtré et concentré ; le résidu pâteux obtenu est dissous dans 2 litres d'acétone puis filtré après refroidissement à 5° C. Le produit cristallisé recueilli est séché.

On obtient 105 g (rendement 50 %) de N-2-éthylhexyloxycarbonyl-N-méthyl-D-glucamine.

Point de fusion 74,2° C.

### ANALYSE ELEMENTAIRE :

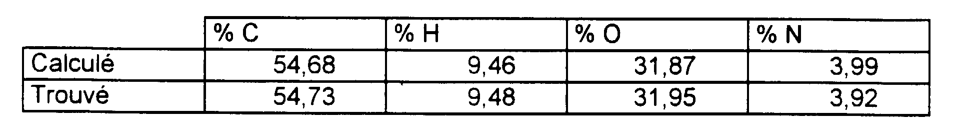

### Exemple 2 : Préparation de la N-2-hexyldicyloxycarbonyl-N-méthyl-D-glucamine

Le composé est préparé selon le même mode opératoire de l'exemple 1, en utilisant:
- 70,2 g (0,36 mole) de N-méthyl-D-glucamine dissous dans un mélange de 60 ml d'eau et de 80 ml de tétrahydrofurane,
- 20,96 g (1,44 mole) d'hydrogénocarbonate de sodium,
- 109,62 g (0,36 mole) de chloroformiate de 2-hexyldécyle,

Le résidu solide issu du mélange réactionnel est recristallisé dans un litre d'acétone puis une seconde fois dans 0,5 litre d'acétone. Après refroidissement à 0° C pendant 12 heures, le produit cristallisé est récupéré et séché.

On obtient 100 g (rendement 60 %) de N-2-hexyldécyloxycarbonyl-N-méthyl-D-glucamine.

Point de fusion : 70,6° C.

### ANALYSE ELEMENTAIRE :

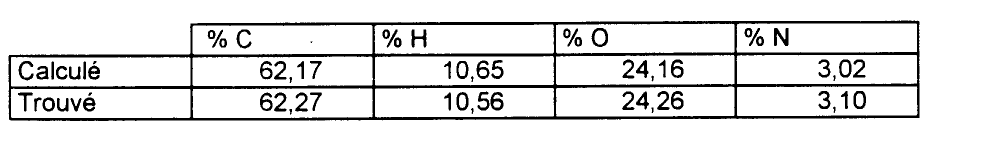

### Exemple 3 : Préparation de la N-2-butyloctyloxycarbonyl-N-méthyl-D-glucamine

Le composé est préparé selon le même mode opératoire de l'exemple 1, en utilisant :
- 78 g (0,4 mole) de N-méthyl-D-glucamine
- 134,4 g (1,6 mole) de bicarbonate de soude
- 99,4 g (0,4 mole) de chloroformiate de 2-butyloctyle.

On obtient 62 g de N-2-butyloctyloxycarbonyl-N-méthyl-D-glucamine sous forme d'une poudre blanche dont le point de fusion est de 77° C.

### ANALYSE ELEMENTAIRE :

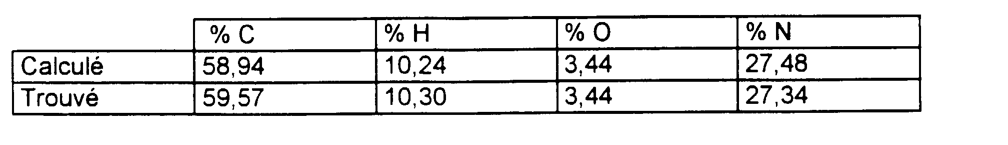

### Exemple 4 : Préparation de la N-2-décyltétradécyloxycarbonyl-N-méthyl-D-glucamine

Le composé est préparé selon le même mode opératoire de l'exemple 1, en utilisant :
- 39 g (0,2 mole) de N-méthyl-D-glucamine,
- 67,2 g (0,8 mole) de bicarbonate de soude,
- 83,3 g (0,2 mole) de chloroformiate de 2-décyltétradécyle.

On obtient 58 g de N-2-décyltétradécyloxycarbonyl-N-méthyl-D-glucamine sous la forme d'une poudre blanche dont le point de fusion est de 63° C.

### ANALYSE ELEMENTAIRE :

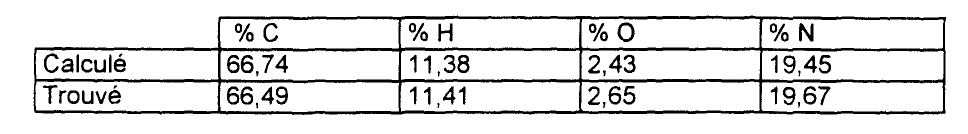

### Exemple 5 : Préparation de la N-2-dodécylhexadécyloxycarbonyl-N-méthyl-D-glucamine

Le composé est préparé selon le même mode opératoire de l'exemple 1, en utilisant :
- 78 g (0,4 mole) de N-méthyl-D-glucamine,
- 134,4 g (1,6 mole) de bicarbonate de soude,
- 189 g (0,4 mole) de chloroformiate de 2-dodécylhexadécyle

On obtient 163 g de N-2-dodécylhexadécyloxycarbonyl-N-méthyl-D-glucamine sous forme d'une poudre blanche dont le point de fusion est de 54° C.

### ANALYSE ELEMENTAIRE :

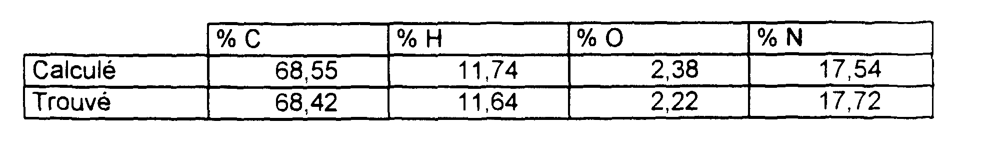

### Exemple 6 : Préparation de la N-2-décyltétradécyloxycarbonyl-D-glucamine

Dans un réacteur de 1000 ml, on solubilise 36,2 g (0,2 mole) de D-glucamine dans 100 ml d'eau. On ajoute 135 ml de tétrahydrofurane puis 67,2 g (0,8 mole) de bicarbonate de soude. Sous agitation, on porte le mélange à -2° C puis on additionne goutte à goutte en 150 minutes 83,3 g (0,2 mole) de chloroformiate de 2-décyltétradécyle. A la fin de l'addition, on laisse le mélange deux heures à 0° C puis on le laisse revenir progressivement en deux heures également à 25° C.

On élimine sur fritté l'insoluble puis on concentre à sec à l'évaporateur rotatif.

Le résidu est recristallisé dans un litre d'acétone, cette opération est renouvelée une fois dans un litre d'acétone puis une fois dans un litre de mélange acétate d'éthyle/ acétone (1/1 vol/vol).

On obtient 61 g de N-2-décyltétradécyloxycarbonyl-D-glucamine sous forme d'une poudre blanche dont le point de fusion est de 82° C.

### ANALYSE ELEMENTAIRE :

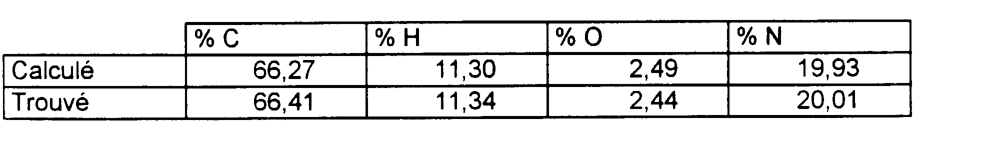

### Exemple 7 : Préparation de la N-2-hexyldécyloxycarbonyl-D-glucamine

Le composé est préparé selon le même mode opératoire de l'exemple 6, en utilisant :
- 46,8 g (0,24 mole) de D-glucamine,
- 80,6 g (0,96 mole) de bicarbonate de soude,
- 73,08 g (0,24 mole) de chloroformiate de 2-hexyldécyle.

On obtient 70 g de N-2-hexyldécyloxycarbonyl-D-glucamine sous forme d'une poudre blanche dont le point de fusion est de 81° C.

### Exemple 8: Préparation du 3-N-(2-décyltétradécyloxycarbonyl)-amino-1,2-propanediol

Le composé est préparé selon le même mode opératoire de l'exemple 6, en utilisant:
- 18,2 g (0,2 mole) de 3-amino -1,2-propanediol,
- 67,2 g (0,8 mole) de bicarbonate de soude,
- 83,3 g (0,2 mole) de chloroformiate de 2-décyltétradécyle.

On obtient 95 g d'une huile ambrée qui est purifiée sur silice à l'aide de mélanges dichlorométhane/méthanol (de 10/0 à 9/1 vol/vol).

On obtient 80 g d'une cire blanche dont la pureté a été controlée par chromatographie sur couche mince (éluant dichlorométhane/méthanol: 95/5 vol/vol Rf = 0,34)

### ANALYSE ELEMENTAIRE :

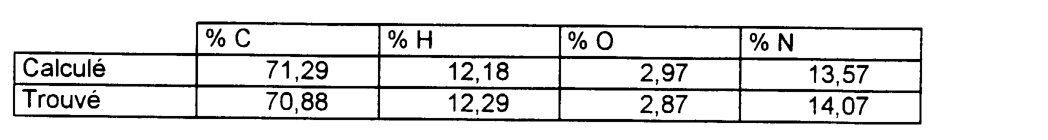

### Exemple 9 : Préparation du 2-N-(2-décyltétradécyloxycarbonyl)-amino-2-hydroxyméthyl-1,3-propanediol

Le composé est préparé selon le même mode opératoire de l'exemple 6, en utilisant :
- 24,2 g (0,2 mole) de tris(hydroxyméthyl) aminométhane,
- 67,2 g (0,8 mole) de bicarbonate de sodium,
- 83,3 g (0,2 mole) de chloroformiate de 2-décyltétradécyle.

On obtient, après réaction et purification sur silice (éluant heptane/acétate d'éthyle : 7/3 puis 6/4 vol/vol), 40 g d'une cire blanche dont le point de fusion est de 51° C et dont la pureté a été vérifée par chromatographie sur couche mince (dichlorométhane/méthanol : 9/1 vol/vol; Rf = 0,45).

### ANALYSE ELEMENTAIRE :

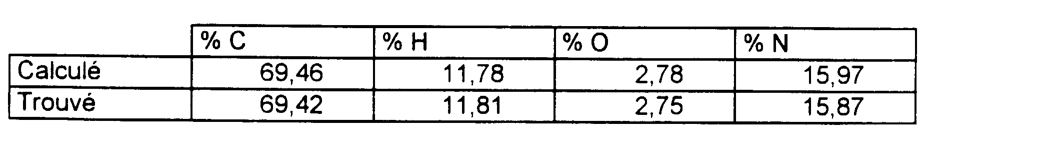

### Exemple 10 : Préparation du 2-N-(2-hexyldécyloxycarbonyl)-amino-1-éthanol

### 1ère étape :

Dans un réacteur de deux litres, on solubilise 105,3 g de carbonyldiimidazole (0,65 mole) dans 1200 ml de dichlorométhane.

On ajoute ensuite goutte à goutte en 1 heure à 20° C 133,38 g (0,55 mole) de 2-hexyldécanol. On laisse agiter 4 heures à 20° C.

Le mélange réactionnel est versé dans une ampoule à décanter et lavé 4 fois avec 200 ml d'eau. La phase organique est séchée sur sulfate de sodium puis concentrée à sec.

On obtient 173 g de 2-hexyldécyloxycarbonyl imidazole sous la forme d'une huile jaune.

### 2ème étape :

Dans un réacteur de 1 litre, on solubilise 12,2 g (0,2 mole) d'aminoéthanol dans 400 ml de tétrahydrofurane anhydre. On ajoute à la température de 20° C une solution de 73,9 g (0,22 mole) de 2-hexyldécyloxycarbonylimidazole (préparé à la 1ère étape) dans 300 ml de tétrahydrofuranne en 90 minutes.

On poursuit l'agitation pendant 24 heures à 20° C puis on évapore à sec. On obtient 88 g d'une huile qui est solubilisée dans 500 ml d'acétate d'éthyle puis lavée par trois fois 100 ml d'eau. Les phases organiques sont regroupées, séchées sur sulfate de sodium puis évaporées à sec.

On obtient 71 g d'une huile ambrée qui est purifiée sur silice (éluant heptane/ acétate d'éthyle 9/1 puis 8/2 vol/vol) pour conduire à 35 g d'une huile incolore dont la pureté a été contrôlée par chromatographie sur couche mince (éluant acétate d'éthyle/heptane 5/5 vol/vol ; Rf = 0,55).

### ANALYSE ELEMENTAIRE :

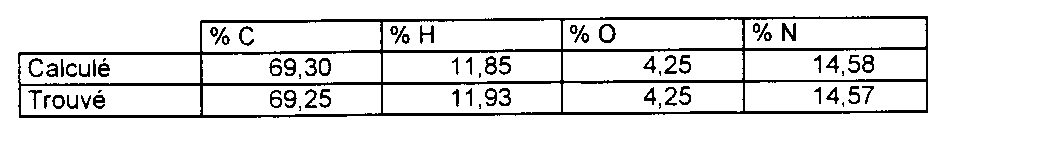

### Exemple 11 : Préparation du 2-N-(2-décyltétradécyloxycarbonyl)-amino-1-éthanol

### 1ère étape :

On opère dans les mêmes conditions qu'à la première étape de l'exemple 10, en utilisant :
- 177 g (0,5 mole) de 2-décyltétradécanol,
- 89,1 g (0,55 mole) de carbonyldiimidazole,
- 1200 ml de dichlorométhane.

On obtient 230 g de 2-décyltétradécyloxycarbonyl imidazole sous forme d'une huile ambrée.

### 2ème étape :

On opère dans les mêmes conditions qu'à la deuxième étape de l'exemple 10, en utilisant :
- 89,2 g (0,2 mole) de 2-décyltétradécyloxycarbonyl imidazole,
- 13,42 g (0,22 mole) d'aminoéthanol,
- 500 ml de tétrahydrofurane.

On obtient 73 g d'une cire blanche dont le point de fusion est de 48° C.

### ANALYSE ELEMENTAIRE :

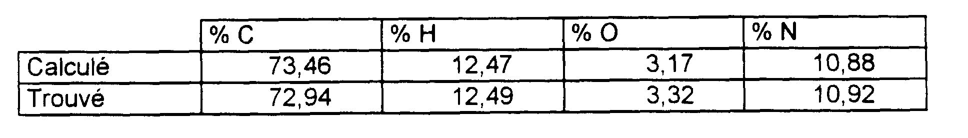

### Exemple 12: Shampooing

- Alkyl (C₉/C₁₀/C₁₁ - 20/40/40) polyglucoside (1,4) vendu sous la dénomination "APG 300" par la Société HENKEL à 50 g % en matière active 15 g MA
- Composé de l'exemple 2 0,1 g
- Conservateurs qs
- Eau qsp 100 g

Le pH est ajusté à 7 avec de l'acide chlorhydrique.

On obtient une solution limpide fluide.

### Exemple 13: Shampooing

- Lauryléther carboxylate de sodium (C₁₂/C₁₄ 70/30) oxyéthyléné à 4,5 moles d'oxyde d'éthylène en solution aqueuse à 22 % en poids vendu sous la dénomination "AKYPOSOFT 45 NV" par la Société CHEMY 7,7 g MA
- Lauryléther sulfate de sodium (C₁₂/C₁₄ 70/30) oxyéthyléné à 2,2 moles d'oxyde d'éthylène en solution aqueuse à 82 % en poids vendu sous la dénomination "EMPICOL ESB/3FL" par la Société ALBRIGHT & WILSON SAINT-MIHIEL 8,5 g MA
- Monoisopropanolamide d'acide de coprah 3 g
- Ether de myristyl glycol et de suif oxyéthyléné à 60 moles d'oxyde d'éthylène vendu sous la dénomination "ELFACOS GT 2825" par la Société AKZO 0,5 g
- Composé de l'exemple 8 0,8 g
- Conservateur qs
- Eau qsp 100 g

Le pH est ajusté à 7 avec de la soude.

On obtient un liquide épaissi limpide.

### Exemple 14: Shampooing

- Lauryléther carboxylate de sodium (C₁₂/C₁₄ 70/30) oxyéthyléné à 4,5 moles d'oxyde d'éthylène en solution aqueuse à 22 % en poids vendu sous la dénomination "AKYPOSOFT 45 NV" par la Société CHEMY 15 g MA
- Lauryléther sulfate de sodium (C₁₂/C₁₄ 70/30) oxyéthyléné à 2,2 moles d'oxyde d'éthylène en solution aqueuse à 82 % en poids vendu sous la dénomination "EMPICOL ESB/3FL" par la Société ALBRIGHT & WILSON SAINT-MIHIEL 8 g MA
- Monoisopropanolamide d'acide de coprah 2 g
- Ether de myristyl glycol et de suif oxyéthyléné à 60 moles d'oxyde d'éthylène vendu sous la dénomination "ELFACOS GT 2825" par la Société AKZO 1 g
- Composé de l'exemple 11 0,4 g
- Conservateur 100 g

Le pH est ajusté à 7 avec de la soude.

On obtient un gel transparent fluide.

### Exemple 15: Shampooing

- Lauryléther carboxylate de sodium 15 g MA
- Mélange de cocoylaminopropylbétaïne et de mono-laurate de glycérol (25/5) en solution aqueuse à 30 % en poids vendu sous la dénomination "TEGOBETAÏNE HS" par la société GOLDSCHMIDT 4g MA
- Di-oléate de polyéthylène glycol (à 55 moles d'oxyde d'éthylène) et de propylène glycol/eau (40/40/20) vendu sous la dénomination "ANTIL 141 LIQUID" par la société GOLDSCHMIDT 1,2 g MA
- Composé de l'exemple 3 0,3 g
- Conservateur qs
- Eau qsp 100 g

Le pH est ajusté à 6, 8 avec de la soude.

On obtient un liquide épaissi limpide.

On procède au shampooing d'une chevelure avec les compositions des exemples 12 à 15. Après séchage, les cheveux sont lisses, brillants et facile à démêler.

### Exemple 16 : Après-shampooing

- Chlorure de béhényl triméthyl ammonium à 80 % en poids dans un mélange eau/isopropanol (15/85) vendu sous la dénomination "CATINAL DC 80" par la Société TOHO 2 g MA
- Composé de l'exemple 4 0,5 g
- Conservateurs qs
- Eau qsp 100 g

On obtient une solution fluide opaque.

### Exemple 17 : Après-shampooing

- Chlorure de cétyl triméthyl ammonium en solution aqueuse à 25 % en poids vendu sous la dénomination "DEHYQUART A" par la Société HENKEL 0,5 g MA
- Composé de l'exemple 5 0,5 g
- Conservateurs qs
- Eau qsp 100 g

Le pH est ajusté à 5,5 par la soude.

### Exemple 18 : Après-shampooing

- Chlorure de béhényl triméthyl ammonium à 80 % en poids dans un mélange eau/isopropanol (15/85) vendu sous la dénomination "CATINAL DC 80" par la Société TOHO 5 g MA
- Composé de l'exemple 7 0,25 g
- Conservateurs qs
- Eau qsp 100 g

On obtient une solution fluide laiteuse.

Les après-shampooings des exemples 16 à 18 sont appliqués sur des cheveux mouillés après un simple shampooing. Après rinçage à l'eau, puis séchage, les cheveux sont lisses, légers et gainés. La coiffure présente un excellent maintien.

### Exemple 19 : Lotion non rincée

- Monolaurate de sorbitane oxyéthyléné à 20 moles d'oxyde d'éthylène 0,5 g
- Composé de l'exemple 6 0,001 g
- Conservateurs qs
- Eau qsp 100 g

Le pH est ajusté à 7 avec de la soude.

On obtient une lotion limpide fluide.

### Exemple 20 : Lotion non rincée

- Méthylsulfate de 1-méthyl 2-suif 3 suifamidoéthyl imidazolinium en solution à 75 % en poids dans le propylène glycol vendu sous la dénomination "REWOQUAT W 75 PG" par la Société REWO 0,2 g MA
- Composé de l'exemple 9 0,005 g
- Conservateur qs
- Eau qsp 100 g

Le pH est ajusté à 5 avec de la soude.

On obtient une lotion fluide opalescente.

On applique les lotions des exemples 19 et 20 sur des cheveux mouillés après un simple shampooing. Sans rincer les cheveux, on les sèche puis on les coiffe. Les cheveux sont uniformément lisses, légers, gainés et facile à démêler.

## Revendications

1. Composés caractérisés par le fait qu'ils répondent à la formule générale (I) suivante: 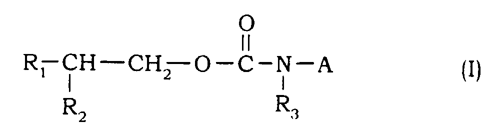 dans laquelle:
- R₁ représente un radical alkyle ou alkylène ayant de 4 à 18 atomes de carbone,
- R₂ représente un radical alkyle ou alkylène ayant de 2 à 16 atomes de carbone,
- R₃ représente un atome d'hydrogène ou un radical alkyle ayant de 1 à 6 atomes de carbone,
- A représente un groupement hydrophile non-ionique.

2. Composés selon la revendication 1, caractérisés par le fait que R₁ représente un radical alkyle ayant de 4 à 14 atomes de carbones, R₂ représente un radical alkyle ayant de 2 à 12 atomes de carbone et R₃ représente un atome d'hydrogène ou un radical méthyle.

3. Composés selon les revendications 1 et 2, caractérisés par le fait que A représente un radical 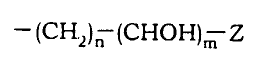 dans lequel n représente un nombre entier égal à 0 ou 1, m représente un nombre entier compris entre 0 et 5, et Z est un radical alkyle monohydroxylé ou polyhydroxylé ayant de 1 à 4 atomes de carbone.

4. Composés selon la revendication 3, caractérisés par le fait que Z représente un radical hydroxylé choisi parmi le groupe comprenant les radicaux suivants:
-CH₂OH -CH₂-CH₂OH -CH(CH₂OH)₂
-C(CH₂OH)₃

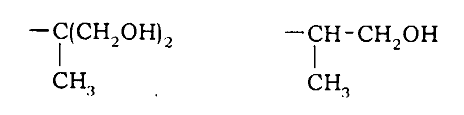

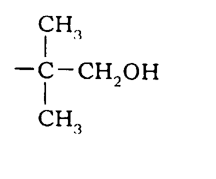

5. Composés selon la revendication 4, caractérisés par le fait que les composés sont choisis parmi :
- le N-2-éthylhexyloxycarbonyl-N-méthyl-D-glucamine
- le N-2-hexyldécyloxycarbonyl-N-méthyl-D-glucamine
- le N-2-butyloctyloxycarbonyl-N-méthyl-D-glucamine
- le N-2-décyltétradécyloxycarbonyl-N-méthyl-D-glucamine
- le N-2-dodécylhexadécyloxycarbonyl-N-méthyl-D-glucamine
- le N-2-décyltétradécyloxycarbonyl-D-glucamine
- le N-2-hexyldécyloxycarbonyl-D-glucamine
- le 3-N-(2-décyltétradécyloxycarbonyl)-amino-1,2-propanediol
- le 2-N-(2-décyltétradécyloxycarbonyl)-amino-2-hydroxyméthyl-1,3-propanediol
- le 2-N-(2-hexyldécyloxycarbonyl)-amino-1-éthanol
- le 2-N-(2-décyltétradécyloxycarbonyl)-amino-1-éthanol

6. Procédé de préparation des composés de formule générale (I) suivante : 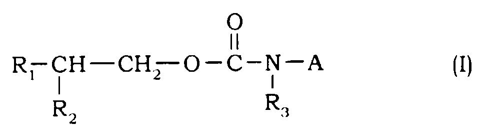 dans laquelle:
- R₁ représente un radical alkyle ou alkylène ayant de 4 à 18 atomes de carbone,
- R₂ représente un radical alkyle ou alkylène ayant de 2 à 16 atomes de carbone,
- R₃ représente un atome d'hydrogène ou un radical alkyle ayant de 1 à 6 atomes de carbone,
- A représente un groupement hydrophile non-ionique,
caractérisé par le fait qu'il consiste à faire réagir dans un solvant inerte un amino alcool de formule R₃-NH-A avec un composé de formule (II) : 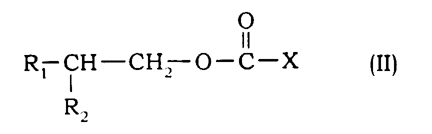 R₁, R₂, R₃, A ayant les même significations que celles données à la revendication 1 et X représente un atome d'halogène ou un radical dérivé d'un azole.

7. Procédé selon la revendication 6 caractérisé par le fait que X est un atome de chlore ou un radical dérivé d'un imidazole.

8. Procédé selon les revendications 6 ou 7, caractérisé par le fait que A représente un radical 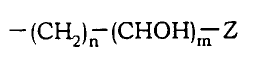 dans lequel n représente un nombre entier égal à 0 ou 1, m représente un nombre entier compris entre 0 et 5, et Z est un radical monohydroxylé ou polyhydroxylé ayant de 1 à 4 atomes de carbone.

9. Procédé selon l'une quelconque des revendications 6 à 8, caractérisé par le fait que Z représente un radical hydroxylé choisi parmi le groupe comprenant les radicaux suivants:
-CH₂OH -CH₂-CH₂OH -CH(CH₂OH)₂
-C(CH₂OH)₃

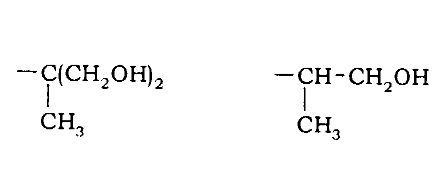

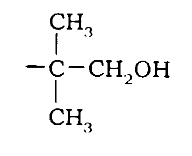

10. Composition cosmétique caractérisée par le fait qu'elle comprend au moins un composé de formule (I) tel que défini dans les revendications 1 à 5.

11. Composition selon la revendication 10, caractérisée par le fait qu'elle se présente sous forme de lotion aqueuse ou hydroalcoolique, de gel, de crème, d'émulsion, de dispersion vésiculaire, de mousse, de spray.

12. Composition cosmétique selon les revendications 10 ou 11, caractérisée par le fait que le composé de formule (I) est présent à une concentration comprise entre 0,001 et 15 % en poids par rapport au poids total de la composition.

13. Composition cosmétique selon l'une quelconque des revendications 10 à 12, caractérisée par le fait que le composé de formule (I) est présent à une concentration comprise entre 0,1 et 10 % en poids par rapport au poids total de la composition.

14. Composition selon l'une quelconque des revendications 10 à 13, caractérisée en ce qu'il s'agit d'une composition capillaire.

15. Composition selon la revendication 14, caractérisée par le fait qu'elle se présente sous forme de shampooing, d'après-shampooing à rincer ou non, de composition pour permanente, défrisage, coloration ou décoloration, ou encore sous forme de composition à rincer, à appliquer avant ou après une coloration, une permanente ou un défrisage ou encore entre les deux étapes d'une permanente ou d'un défrisage.

16. Composition cosmétique selon l'une quelconque des revendications 10 à 15. caractérisée par le fait qu'elle contient au moins un additif cosmétique.

17. Utilisation d'une composition selon l'une quelconque des revendications 10 à 16 pour le traitement des cheveux.

18. Procédé de traitement des cheveux consistant à appliquer sur les cheveux une composition selon l'une quelconque des revendications 10 à 16.

## Claims

1. Compounds characterized in that they correspond to the following general formula (I): 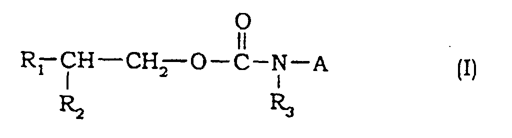 in which:
- R₁ represents an alkyl or alkylene radical having from 4 to 18 carbon atoms,
- R₂ represents an alkyl or alkylene radical having from 2 to 16 carbon atoms,
- R₃ represents a hydrogen atom or an alkyl radical having from 1 to 6 carbon atoms,
- A represents a non-ionic hydrophilic group.

2. Compounds according to Claim 1, characterized in that R₁ represents an alkyl radical having from 4 to 14 carbon atoms, R₂ represents an alkyl radical having from 2 to 12 carbon atoms and R₃ represents a hydrogen atom or a methyl radical.

3. Compounds according to Claims 1 and 2, characterized in that A represents a radical
-(CH₂)ₙ-(CHOH)ₘ-Z
in which n represents an integer equal to 0 or 1, m represents an integer between 0 and 5 and Z is a monohydroxylated or polyhydroxylated alkyl radical having from 1 to 4 carbon atoms.

4. Compounds according to Claim 3, characterized in that Z represents a hydroxylated radical chosen from the group comprising the following radicals:
-CH₂OH -CH₂-CH₂OH -CH(CH₂OH)₂
-C(CH₂OH)₃

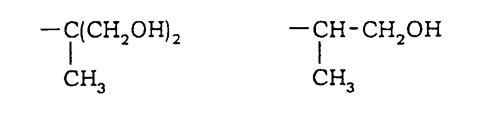

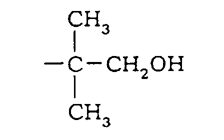

5. Compounds according to Claim 4, characterized in that the compounds are chosen from:
- N-2-ethylhexyloxycarbonyl-N-methyl-D-glucamine
- N-2-hexyldecyloxycarbonyl-N-methyl-D-glucamine
- N-2-butyloctyloxycarbonyl-N-methyl-D-glucamine
- N-2-decyltetradecyloxycarbonyl-N-methyl-D-glucamine
- N-2-dodecylhexadecyloxycarbonyl-N-methyl-D-glucamine
- N-2-decyltetradecyloxycarbonyl-D-glucamine
- N-2-hexyldecyloxycarbonyl-D-glucamine
- 3-[N-(2-decyltetradecyloxycarbonyl)amino]-1,2-propanediol
- 2-[N-(2-decyltetradecyloxycarbonyl)amino]-2-hydroxymethyl-1,3-propanediol
- 2-[N-(2-hexyldecyloxycarbonyl)amino]-1-ethanol
- 2-[N-(2-decyltetradecyloxycarbonyl)amino]-1-ethanol.

6. Process for the preparation of the compounds of following general formula (I): 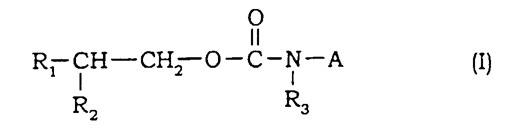 in which:
- R₁ represents an alkyl or alkylene radical having from 4 to 18 carbon atoms,
- R₂ represents an alkyl or alkylene radical having from 2 to 16 carbon atoms,
- R₃ represents a hydrogen atom or an alkyl radical having from 1 to 6 carbon atoms,
- A represents a non-ionic hydrophilic group,
characterized in that it consists in reacting, in an inert solvent, an aminoalcohol of formula R₃-NH-A with a compound of formula (II): 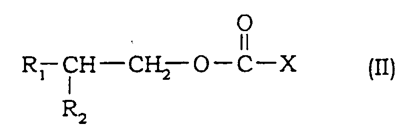 R₁, R₂, R₃ and A having the same meanings as those given in Claim 1 and X represents a halogen atom or a radical derived from an azole.

7. Process according to Claim 6, characterized in that X is a chlorine atom or a radical derived from an imidazole.

8. Process according to Claim 6 or 7, characterized in that A represents a radical
-(CH₂)ₙ-(CHOH)ₘ-Z
in which n represents an integer equal to 0 or 1, m represents an integer between 0 and 5 and Z is a monohydroxylated or polyhydroxylated radical having from 1 to 4 carbon atoms.

9. Process according to any one of Claims 6 to 8, characterized in that Z represents a hydroxylated radical chosen from the group comprising the following radicals:
-CH₂OH -CH₂-CH₂OH -CH(CH₂OH)₂
-C(CH₂OH)₃

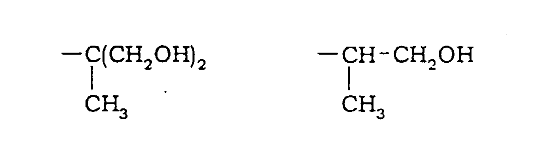

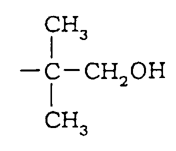

10. Cosmetic composition, characterized in that it comprises at least one compound of formula (I) as defined in Claims 1 to 5.

11. Composition according to Claim 10, characterized in that it is provided in the form of an aqueous or aqueous/alcoholic lotion, of a gel, of a cream, of an emulsion, of a vesicular dispersion, of a foam or of a spray.

12. Cosmetic composition according to Claim 10 or 11, characterized in that the compound of formula (I) is present at a concentration of between 0.001 and 15% by weight with respect to the total weight of the composition.

13. Cosmetic composition according to any one of Claims 10 to 12, characterized in that the compound of formula (I) is present at a concentration of between 0.1 and 10% by weight with respect to the total weight of the composition.

14. Composition according to any one of Claims 10 to 13, characterized in that it is a hair composition.

15. Composition according to Claim 14, characterized in that it is provided in the form of a shampoo, of a conditioner, which is or is not to be rinsed, of a permanent wave, hair straightening, dyeing or bleaching composition or alternatively in the form of a composition to be rinsed, to be applied before or after a dyeing, a permanent wave or a hair straightening or alternatively between the two stages of a permanent wave or of a hair straightening.

16. Cosmetic composition according to any one of Claims 10 to 15, characterized in that it contains at least one cosmetic additive.

17. Use of a composition according to any one of Claims 10 to 16 in hair treatment.

18. Hair treatment process consisting in applying a composition according to any one of Claims 10 to 16 to hair.

## Patentansprüche

1. Verbindungen, die dadurch gekennzeichnet sind, daß sie die folgende allgemeine Formel (I) 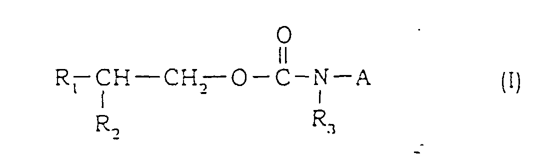 aufweisen, worin bedeuten:
- R₁ eine Alkyl- oder Alkylengruppe mit etwa 4 bis 18 Kohlenstoffatomen,
- R₂ eine Alkyl- oder Alkylengruppe mit etwa 2 bis 16 Kohlenstoffatomen,
- R₃ ein Wasserstoffatom oder eine Alkylgruppe mit etwa 1 bis 6 Kohlenstoffatomen und
- A eine nichtionische hydrophile Gruppe.

2. Verbindungen nach Anspruch 1, dadurch gekennzeichnet, daß R₁ eine Alkylgruppe mit 4 bis 14 Kohlenstoffatomen, R₂ eine Alkylgruppe mit 2 bis 12 Kohlenstoffatomen und R₃ ein Wasserstoffatom oder eine Methylgruppe darstellt.

3. Verbindungen nach den Ansprüchen 1 und 2, dadurch gekennzeichnet, daß A eine Gruppe
- (CH₂)ₙ-(CHOH)ₘ-Z
ist, in der n die ganze Zahl 0 oder 1, m eine ganze Zahl von 0 bis 5 und Z eine Alkylgruppe mit 1 bis 4 Kohlenstoffatomen, die eine oder mehrere Hydroxygruppen aufweist, darstellt.

4. Verbindungen nach Anspruch 3, dadurch gekennzeichnet, daß Z eine hydroxygruppenhaltige Gruppe darstellt, die ausgewählt ist unter:
-CH₂OH -CH₂-CH₂OH -CH(CH₂OH)₂
-C(CH₂OH)₃

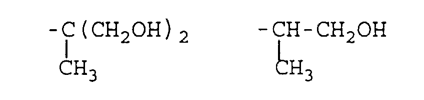

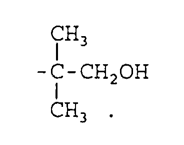

5. Verbindung nach Anspruch 4, dadurch gekennzeichnet, daß sie ausgewählt sind unter:
- *N*-2-Ethylhexyloxycarbonyl-*N*-methyl-D-glucamin,
- *N*-2-Hexyldecyloxycarbonyl-*N*-methyl-D-glucamin,
- *N*-2-Butyloctyloxycarbonyl-*N*-methyl-D-glucamin,
- *N*-2-Decyltetradecyloxycarbonyl-*N*-methyl-D-glucamin,
- *N*-2-Dodecylhexadecyloxycarbonyl-*N*-methyl-D-glucamin,
- *N*-2-Decyltetradecyloxycarbonyl-D-glucamin,
- *N*-2-Hexyldecyloxycarbonyl-D-glucamin,
- 3-*N*-(2-Decyltetradecyloxycarbonyl)-amino-1,2-propandiol,
- 2-*N*-(2-Decyltetradecyloxycarbonyl)-amino-2-hydroxymethyl-1,3-propandiol,
- 2-*N*-(2-Hexyldecyloxycarbonyl)-amino-1-ethanol, und
- 2-*N*-(2-Decyltetradecyloxycarbonyl)-amino-1-ethanol.

6. Verfahren zur Herstellung der Verbindungen der folgenden allgemeinen Formel (I) 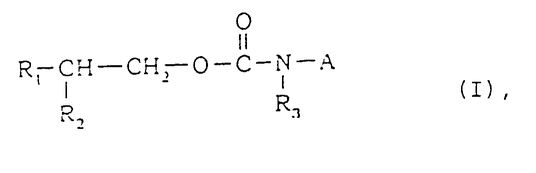 worin bedeuten:
- R₁ eine Alkyl- oder Alkylengruppe mit etwa 4 bis 18 Kohlenstoffatomen,
- R₂ eine Alkyl- oder Alkylengruppe mit etwa 2 bis 16 Kohlenstoffatomen,
- R₃ ein Wasserstoffatom oder eine Alkylgruppe mit etwa 1 bis 6 Kohlenstoffatomen und
- A eine nichtionische hydrophile Gruppe.
dadurch gekennzeichnet, daß
es darin besteht, in einem inerten Lösungsmittel einen Aminoalkohol der Formel R₃-NH-A mit einer Verbindung der Formel (II) 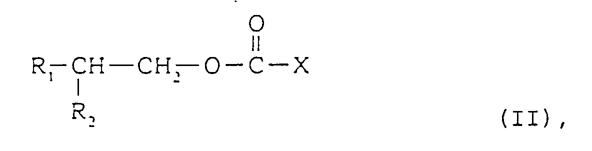 umzusetzen, worin R₁, R₂, R₃ und A dasselbe wie in Anspruch 1 bedeuten und X ein Halogenatom oder eine von einem Azol abgeleitete Gruppe darstellt.

7. Verfahren nach Anspruch 6, dadurch gekennzeichnet, daß X ein Chloratom oder eine von einem Imidazol abgeleitete Gruppe ist.

8. Verfahren nach Anspruch 6 oder 7, dadurch gekennzeichnet, daß A eine Gruppe
-(CH₂)ₙ-(CHOH)ₘ-Z
ist, worin n die ganze Zahl 0 oder 1, m eine ganze Zahl von 0 bis 5 und Z eine Gruppe mit 1 bis 4 Kohlenstoffatomen, die eine oder mehrere Hydroxygruppen aufweist, darstellt.

9. Verfahren nach einem der Ansprüche 6 bis 8, dadurch gekennzeichnet, daß Z eine hydroxygruppenhaltige Gruppe darstellt, die ausgewählt wird unter
-CH₂OH -CH₂-CH₂OH -CH(CH₂OH)₂
-C(CH₂OH)₃

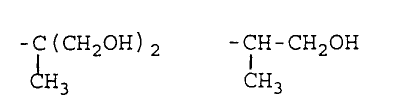

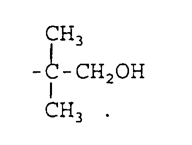

10. Kosmetische Zusammensetzung, dadurch gekennzeichnet, daß sie mindestens eine Verbindung der Formel (I), die wie in den Ansprüchen 1 bis 5 definiert ist, enthält.

11. Kosmetische Zusammensetzung nach Anspruch 10, dadurch gekennzeichnet, daß sie in Form einer wäßrigen oder wäßrig-alkoholischen Lotion, eines Gels, einer Creme, einer Emulsion, einer Vesikeldispersion, eines Schaums oder eines Sprays vorliegt.

12. Kosmetische Zusammensetzung nach Anspruch 10 oder 11, dadurch gekennzeichnet, daß die Verbindung der Formel (I) in einer Konzentration von 0,001 bis 15 Gew.-%, bezogen auf das Gesamtgewicht der Zusammensetzung, enthalten ist.

13. Kosmetische Zusammensetzung nach einem der Ansprüche 10 bis 12, dadurch gekennzeichnet, daß die Verbindung der Formel (I) in einer Konzentration von 0,1 bis 10 Gew.-%, bezogen auf das Gesamtgewicht der Zusammensetzung, enthalten ist.

14. Zusammensetzung nach einem der Ansprüche 10 bis 13, dadurch gekennzeichnet, daß es sich um eine Zusammensetzung für die Haarbehandlung handelt.

15. Zusammensetzung nach Anspruch 14, dadurch gekennzeichnet, daß sie in Form eines Haarwaschmittels, eines nach der Haarwäsche anzuwendenden Haarpflegemittels, das ausgespült wird oder im Haar verbleibt, einer Zusammensetzung für die Dauerwellverformung, die Entkräuselung, die Färbung oder Entfärbung oder in Form einer Zusammensetzung, die ausgespült wird und vor oder nach einer Färbung, einer Dauerwellverformung oder eine Entkräuselung oder auch zwischen den beiden Schritten einer Dauerwellverformung oder Entkräuselung aufgetragen wird, vorliegt.

16. Kosmetische Zusammensetzung nach einem der Ansprüche 10 bis 15, dadurch gekennzeichnet, daß sie mindestens einen kosmetischen Hilfsstoff enthält.

17. Verwendung einer Zusammensetzung nach einem der Ansprüche 10 bis 15 für die Haarbehandlung.

18. Verfahren zur Haarbehandlung, das darin besteht, auf die Haare eine Zusammensetzung nach einem der Ansprüche 10 bis 16 anzuwenden.
